# EUROPÄISCHE PATENTSCHRIFT

(11) **EP 1 032 552 B1**
(45) Veröffentlichungstag und Bekanntmachung des Hinweises auf die Patenterteilung: **06.08.2003**
(21) Anmeldenummer: 98955557.8
(22) Anmeldetag: 09.11.1998
(51) Int. Cl.: C07C 29/149, C07C 33/02, C07C 33/025, C07C 67/54, C07C 69/24, C07C 69/533

(54) **UNGESÄTTIGTE PALMFETTALKOHOLE**
UNSATURATED PALM OIL FATTY ALCOHOLS
ALCOOLS GRAS INSATURES DE PALME

(30) Priorität: 17.11.1997 DE 19750800
(43) Veröffentlichungstag der Anmeldung: 06.09.2000
(73) Patentinhaber: Cognis Deutschland GmbH & Co. KG, 40589 Düsseldorf (DE)
(72) Erfinder: HECK, Stephan, D-50259 Pulheim (DE); KLEIN, Norbert, D-40822 Mettmann (DE); KOMP, Horst-Dieter, D-40764 Langenfeld (DE); BÖHR, Christiane, D-51379 Leverkusen (DE); HÜBNER, Norbert, D-40597 Düsseldorf (DE); WESTFECHTEL, Alfred, D-40724 Hilden (DE)
(86) Internationale Anmeldenummer: EP9807148
(87) Internationale Veröffentlichungsnummer: WO99025673

(56) Entgegenhaltungen:
- DE-A- 4 335 781
- DE-A- 4 425 180

## Beschreibung

Die Erfindung befindet sich auf dem Gebiet der Oleochemikalien und betrifft ein Verfahren zur Herstellung ungesättigter Fettalkohole, bei dem man Palmfettsäuremethylester fraktioniert und die Fraktion ungesättigter langkettiger Methylester dann hydriert.

### Stand der Technik

Ungesättigte Fettalkohole stellen wichtige Zwischenprodukte für eine große Anzahl von Erzeugnissen der chemischen Industrie, wie z.B. für die Herstellung von Tensiden und Hauptpflegeprodukten dar. Eine Übersicht hierzu findet sich beispielsweise von U.Ploog et al. in **Seifen-Öle-Fette-Wachse 109, 225 (1983).** Zu ihrer Herstellung geht man von mehr oder minder ungesättigten Fettsäuremethylestern aus, die beispielsweise in Gegenwart chrom- oder zinkhaltiger Mischkatalysatoren hydriert werden können **[Ullmann's Enzyclopaedie der technischen Chemie, Verlag Chemie, Weinheim, 4. Aufl., Bd. 11, S. 436f].** Stand der Technik ist ein großtechnisches Verfahren, wie es bislang auch bei der Anmelderin durchgeführt worden ist, nach dem tierische Fette und Öle eingesetzt werden, die nach der Hydrierung anfallenden ungesättigten Fettalkohole bei einer Sumpftemperatur von z. B. 220 bis 250°C und einem verminderten Druck von 1 bis 20 mbar - gemessen am Kolonnenkopf - destilliert werden. Da die Herstellung von ungesättigten Fettalkoholen mit hohen Kosten verbunden ist, wurde auf einen möglichst geringen Rohstoffverlust destilliert. Tatsächlich konnte auf diese Weise eine Ausbeute von ca. 90 % der Theorie - und entsprechend ein Verlust von 10 % - erreicht werden, die Produkte zeigten jedoch einen deutlichen Eigengeruch. Ein weiterer Nachteil besteht ferner darin, dass die Fettalkohole des Standes der Technik ein unbefriedigendes Lager- und Kälteverhalten zeigen.

Aus anwendungstechnischen Gründen sind ungesättigte Fettalkohole mit Iodzahlen von 50 bis 80 besonders bevorzugt, da diese einen für die Verwendung in Kosmetikprodukten günstigen Erstarrungspunkt besitzen. Ungesättigte Fettalkohole mit Iodzahlen im oben genannten Bereich basieren derzeit ganz überwiegend auf tierischen Rohstoffen. Der gewünschte Iodzahlbereich wird durch Verschneiden verschiedener Produkte mit unterschiedlichen Iodzahlbereichen eingestellt. Die Einstellung des Iodzahlbereichs durch destillative Verfahren ist nicht möglich, da die Iodzahl bzw. der Iodzahlbereich von Fettalkoholen bzw. Fettsäuren auf tierischer Basis bei der Fraktionierung nahezu konstant bleibt. Tierische Fette haben aber den Nachteil, dass sie sehr heterogen aufgebaut sind. Zum Beispiel enthalten tierische Fette stickstoffhaltige Verbindungen wie Amide oder Steroide, wie z. B. Cholesterin, die direkt oder indirekt für den oben erwähnten unangenehmen Geruch der Produkte verantwortlich sind. Die stickstoffhaltigen Verbindungen können Nebenreaktionen eingehen, was die Produktstabilität, insbesondere die Oxidationsstabilität, verschlechtert und zu verfärbten Produkten führt. Zudem werden vom Verbraucher im Hinblick auf die andauernde BSE-Diskussion Produkte, die unter Verwendung von Rindertalg hergestellt werden äußerst kritisch gesehen. Auf dem Kosmetikmarkt besteht daher ein stetiges Bedürfnis nach immer reineren und qualitativ hochwertigeren Rohstoffen, eine Forderung, die üblicherweise nur durch immer aufwendigere technische Verfahren und zusätzliche Reinigungsschritte zur Verfügung gestellt werden können. Im Fall der ungesättigten Fettalkohole besteht dabei insbesondere das Bedürfnis nach Produkten mit verbesserter Farb- und Geruchsqualität sowie vorteilhafterem Kälteverhalten. Hinzukommt, dass sich in den letzten Jahren das Verbraucherverhalten dahingehend verändert hat, dass die Verbraucher sehr großen Wert auf rein pflanzliche Produkte legen. Die bekannten pflanzlichen Fettalkohole weisen Iodzahlen in dem Bereich unter 20 oder sehr hohe Iodzahlen über 100 auf. Fettalkohole mit Iodzahlen in dem oben genannten anwendungstechnisch besonders bevorzugten Bereich zwischen 20 und 95 sind nicht bekannt. Das Verschneiden der Fettalkohole mit sehr unterschiedlichen Iodzahlen führt nicht zu zufriedenstellenden Produkten.

Aus der deutschen Offenlegungsschrift **DE-A1 4335781** (Henkel) ist ein Verfahren bekannt, bei dem man die in den pflanzlichen Fetten Rohstoffen enthaltenen Triglyceride zunächst durch Druckspaltung in Glycerin und Fettsäuren spaltet und letztere mit Methanol verestert bzw. die Ausgangsstoffe direkt zu den Fettsäuremethylcstern umestert und anschließend die Ester zu den Alkoholen hydriert, wobei entweder die Fettsäuremethylester oder die Hydrierprodukte durch Abnahme einer solchen Menge Vorlauf fraktioniert werden, dass das Endprodukt eine Iodzahl von 20 bis 110 und einen Konjuengehalt von weniger als 4,5 Gew.-% aufweist. Während sich das Verfahren auf pflanzliche Rohstoffe wie Palmöl für die Herstellung von ungesättigten Fettalkoholen im Iodzahlbereich 50 bis 65 problemlos anwenden lässt, werden beim Einsatz von Palmöl zur Erzeugung von ungesättigten Fettalkoholen im Iodzahlbereich von 65 bis 85 überraschenderweise nicht in jeder Hinsicht befriedigende Ergebnisse erzielt.

Die Aufgabe der vorliegenden Erfindung hat folglich darin bestanden, ungesättigte Fettalkohole auf Basis von Palmöl zur Verfügung zu stellen, die Iodzahlen im Bereich von 65 bis 85 aufweisen und gegenüber ungesättigten Fettalkoholen auf tierischer Basis eine größere Oxidationsstabilität und ein gleichwertiges oder besseres Kälteverhalten aufweisen. Gleichzeitig sollten möglichst reine Koppelprodukte erhalten werden.

### Beschreibung der Erfindung

Gegenstand der Erfindung ist ein Verfahren zur Herstellung ungesättigter Palmfettalkohole mit einer Iodzahl im Bereich von 65 bis 85, die im wesentlichen ungesättigte Fettalkohole und Gemische aus gesättigten Fettalkoholen der Formel **(I)** enthalten,

**R**^{**1**}**OH** (I)

in der R¹ für einen gesättigten oder ungesättigten, linearen oder verzweigten Alkylrest mit 14 bis 20 Kohlenstoffatomen steht, bei dem man
(a) Palmfettsäuremethylester in ein überwiegend gesättigtes C₁₆-Destillat und ein überwiegend ungesättigtes C_{16/18}-Sumpfprodukt fraktioniert, und
(b) das Sumpfprodukt unter Erhalt der Doppelbindungen zu den entsprechenden Alkoholen hydriert.

Überraschenderweise wurde gefunden, dass nach dem erfindungsgemäßen Verfahren nun erstmals auch auf Basis von Palmöl ungesättigte Fettalkohole im Iodzahlbereich von 65 bis 85 zugänglich sind, die über eine hohe Farb- und Oxidationsstabilität sowie ein ausgezeichnetes Kälteverhalten verfügen; zudem sind die Produkte praktisch geruchsfrei. Ein weiterer Vorteil besteht darin, dass als Zwischenprodukt eine sehr reine Palmitinsäuremethylesterfraktion gewonnen wird, die separat weiterverarbeitet werden kann.

### Fraktionierung

Die Fraktionierung der Palmfettsäuremethylester kann batchweise oder kontinuierlich bei verringertem Druck durchgeführt werden. Die Beheizung kann beispielsweise durch Heißdampf erfolgen, wobei in der Regel eine Sumpftemperatur von z. B. 220 bis 250°C herrscht. Die eigentliche Fraktionierung findet in einer gepackten Kolonne mit druckverlustarmen Einbauten statt. Als Einbauten kommen beispielsweise geordnete Blechpackungen in Betracht. Weitere Beispiele finden sich in **RÖMPP Chemie Lexikon, Thieme Verlag, Stuttgart, 9. Auflage, Bd. 3, S. 2305 (1990)** unter dem Stichwort "Kolonnen-Einbauten" und in der dort genannten Literatur. Das erforderliche Feinvakuum von 1 bis 20 mbar am Kopf der Kolonne kann beispielsweise mit Hilfe von Wasserringpumpen und vorgeschalteten Dampfstrahlern erzielt werden. Vorzugsweise sollte der Druckabfall über die gesamte Destillationsanlage nicht mehr als 20 mbar betragen. Hierbei wird ein Destillat mit überwiegend gesättigten C₁₆-Anteilen und ein Sumpfprodukt mit überwiegend ungesättigten C₁₆-C₁₈-Anteilen erhalten. Das Gewichtsverhältnis von Destillat und Sumpfprodukt liegt im Bereich von 30:70 bis 35:65.

### Hydrierung

Die anschließende Hydrierung der als Sumpf erhaltenen weitgehend ungesättigten Methylesterfraktion unter Erhalt der Doppelbindungen kann in an sich bekannter Weise durchgeführt werden, d.h. beispielsweise in Gegenwart von handelsüblichen Zink/Chrom-Katalysatoren, bei Temperaturen im Bereich von 250 bis 350°C sowie einem Wasserstoffdruck von 200 bis 275 bar. Der Konjuengehalt der Produkte liegt dabei im Bereich von 6 bis 12 Gew.-%, der Gehalt an Kohlenwasserstoffen unter 3, vorzugsweise unter 1 Gew.-%.

### Gewerbliche Anwendbarkeit

Die nach dem erfindungsgemäßen Verfahren erhaltenen, ungesättigten Palmfettalkohole sind farb- und geruchsarm und weisen ein besonders vorteilhaftes Kälteverhalten auf. Sie eignen sich daher als Rohstoffe zur Herstellung von Wasch-, Spül- und Reinigungsprodukten sowie Produkten zur Haar- und Körperpflege, in denen sie in Mengen von 1 bis 50, vorzugsweise 5 bis 30 Gew.-% - bezogen auf die Mittel - enthalten sein können.

### Beispiel

Ein technischer Palmfettsäuremethylester wurde in einer gepackten Kolonne mit druckverlustarmen Einbauten bei einer Sumpftemperatur von 200°C und einem Kopfvakuum von 20 mbar fraktioniert, wobei als Destillat 30 Gew.-% Palmitinsäuremethylester anfielen, während im Sumpf 70 Gew.-% eines C_{16/18}-Fettsäuremethylestergemisches verblieb, welches eine Iodzahl von 74 aufwies. Das Estergemisch aus dem Sumpf wurde in einen Autoklaven überführt und dort in Gegenwart von handelsüblichen Zink/Chrom-Katalysatoren bei 300°C und 250 bar mit Wasserstoff zum Gemisch der korrespondierenden Alkohole reduziert Das von Methanol befreite Hydrierprodukt wies nach gaschromatographischer und nasschemischer Analyse folgende Kenndaten auf:

**Tabelle 1:**

| **Kenndaten Hydrierprodukt** | | | |
|---|---|---|---|
| **Zusammensetzung** | **Anteil [Flächen-%]** | **Spezifikation** | **Wert** |
| Cetylalkohol | 18,3 | Iodzahl | 74 |
| Palmoleylalkohol | 0,5 | Hydroxylzahl | 212 |
| Margarinylalkohol | 0,6 | Säurezahl | 0,02 |
| Stearylalkohol | 8,5 | Verseifungszahl | 0,4 |
| Oleylalkohol | 61,2 | Hazen-Farbzahl | 10 |
| Linolylalkohol | 3,3 | Konjuengehalt | 6,6 Gew.-% |
| Linolenylalkohol | 6,7 | Erstarrungspunkt | 22,7°C |
| Kohlenwasserstoffe | 0,9 | | |

## Patentansprüche

1. Verfahren zur Herstellung von ungesättigten Palmfettalkoholen mit einer Iodzahl im Bereich von 65 bis 85, die im wesentlichen ungesättigte Fettalkohole und Gemische aus gesättigten Fettalkoholen der Formel **(I)** enthalten,
**R**^{**1**}**OH** (I)
in der R¹ für einen gesättigten oder ungesättigten, linearen oder verzweigten Alkylrest mit 14 bis 20 Kohlenstoffatomen steht, bei dem man
(a) Palmfettsäuremethylester in ein überwiegend gesättigtes C₁₆-Destillat und ein überwiegend ungesättigtes C_{16/18}-Sumpfprodukt fraktioniert, und
(b) das Sumpfprodukt unter Erhalt der Doppelbindungen zu den entsprechenden Alkoholen hydriert.

## Claims

1. A process for the preparation of unsaturated palm oil fatty alcohols having an iodine number in the range from 65 to 85, which essentially comprise unsaturated fatty alcohols and mixtures of saturated fatty alcohols of formula (I)
**R**^{**1**}**OH** (I)
in which R¹ is a saturated or unsaturated, linear or branched alkyl radical having 14 to 20 carbon atoms, in which
(a) palm oil fatty acid methyl esters is fractionated into a predominantly saturated C₁₆ distillate and a predominantly unsaturated C_{16/18} bottom product, and
(b) the bottom product is hydrogenated with retention of the double bonds to give the corresponding alcohols.

## Revendications

1. Procédé de production d'alcools de palme non saturés ayant un indice d'iode dans la plage de 65 à 85, qui renferment essentiellement des alcools gras non saturés et des mélanges à base d'alcools gras saturés de formule (I)
**R**^{**1**}**OH** (I)
dans laquelle R¹ représente un reste alkyle saturé ou non saturé, linéaire ou ramifié, ayant de 14 à 20 atomes de carbone, selon lequel
a) on fractionne un ester méthylique d'acide gras de palme en un distillat principalement saturé en C₁₆ et en un produit indistillable en C₁₆/C₁₈ principalement non saturé et
b) on hydrogène le produit indistillable tout en conservant les doubles liaisons, en alcools correspondants.
